# EUROPÄISCHE PATENTSCHRIFT

(11) **EP 2 279 693 B1**
(45) Veröffentlichungstag und Bekanntmachung des Hinweises auf die Patenterteilung: **14.03.2012**
(21) Anmeldenummer: 10170286.8
(22) Anmeldetag: 21.07.2010
(51) Int. Cl.: A61B 5/107

(54) **Vorrichtung und Verfahren zum Erfassen der Gestalt eines Ohrabschnitts**
Method and device for recording the form of an ear section
Dispositif et procédé de détermination de l'état d'une section d'oreille

(30) Priorität: 28.07.2009 DE 102009034993
(43) Veröffentlichungstag der Anmeldung: 02.02.2011
(73) Patentinhaber: Siemens Medical Instruments Pte. Ltd., Singapore 139959 (SG)
(72) Erfinder: Forster, Frank, 81739, München (DE); Holzner, Rudolf, 84032, Altdorf (DE); Kunz, Martin, 81377, München (DE); Rass, Uwe, 90480, Nürnberg (DE); Schick, Anton, 84149, Velden (DE)
(74) Vertreter: Maier, Daniel Oliver

(56) Entgegenhaltungen:
- WO-A1-2008/058882
- US-A1- 2003 164 952
- US-A1- 2009 018 465

## Beschreibung

Die vorliegende Erfindung betrifft eine Vorrichtung zum Erfassen der Gestalt eines Abschnitts eines menschlichen Ohrs, insbesondere eines Gehörgangs. Darüber hinaus betrifft die vorliegende Erfindung auch ein entsprechendes Verfahren zum Erfassen eines Ohrabschnitts.

Zur Herstellung von individuellen Schalen für In-dem-Ohr-Hörgeräte oder von individuellen Otoplastiken ist es notwendig, die dreidimensionale Gestalt eines individuellen Gehörgangs exakt zu vermessen bzw. zu ermitteln.

Bislang wird die Gestalt eines Gehörgangs typischerweise dadurch erfasst, dass Silikonmaterial in den Gehörgang eingespritzt wird. Das Material härtet nach einigen Minuten aus. Anschließend wird das Material aus dem Gehörgang genommen und gescannt, um 3D-Daten zu erhalten. Diese Prozedur ist jedoch für den Patienten unangenehm und insgesamt arbeitsaufwändig.

Aus der Druckschrift WO2008/092820 ist ein Verfahren bekannt, um strukturiertes Licht zu erzeugen.

Aus der Druckschrift US 2003/0164 952 A1 ist eine gattungsgemäße Vorrichtung zum Erfassen der Gestalt eines Abschnitts eines menschlichen Ohrs bzw. Gehörgangs bekannt. Sie besitzt eine Aufnahmeeinrichtung zum Erfassen einer räumlichen Gestalt mehrer Unterabschnitte des zu erfassenden Abschnitts und zum Erfassen je einer die Position repräsentierenden Größe der Unterabschnitte relativ zu einem vorgegebenen, optischen, natürlichen Merkmal des zu erfassenden Abschnitts. Außerdem ist eine Auswerteeinrichtung zum Gewinnen einer Gestaltsinformation über den zu erfassenden Abschnitt durch Kombination der Gestaltung der Unterabschnitte auf der Basis ihrer erfassten Positionen vorgesehen.

Ähnliche Systeme zum Erfassen der dreidimensionalen Gestalt eines Ohrkanals sind aus den Druckschriften US 2009/0018 465 A1 und WO 2008/0588 82 A1 bekannt.

Die Aufgabe der vorliegenden Erfindung besteht darin, das Herstellen von individuellen Hörgeräteschalen bzw. Otoplastiken zu vereinfachen und komfortabler zu gestalten. Insbesondere soll die Ermittlung der dreidimensionalen Gestalt eines Gehörgangs verbessert werden.

Erfindungsgemäß wird diese Aufgabe gelöst durch eine Vorrichtung nach Anspruch 1.

Darüber hinaus wird erfindungsgemäß bereitgestellt ein Verfahren nach Anspruch 6.

In vorteilhafter Weise ist es durch das Erfassen eines oder mehrerer optischer Merkmale an einem Ohr möglich, mehrere Messaufnahmen aneinander exakt auszurichten und so eine dreidimensionale Gestalt eines Abschnitts des Ohrs und insbesondere des Gehörgangs zu gewinnen. Vor allem muss so nicht mehr ein Ohrabdruck gewonnen werden, indem Material in das Ohr eingespritzt wird.

Die Aufnahmeeinrichtung besitzt eine Lasermessanordnung, mit der ein kegelmantelförmiger Messstrahl erzeugbar ist. Damit lässt sich durch Triangulation ein ringförmiger Unterabschnitt des Ohrs bzw. Gehörgangs messen.

Insbesondere können mit der Laseranordnung gleichzeitig mehrere kegelmantelförmige Messstrahle erzeugbar sein. Damit lassen sich gleichzeitig mehrere Unterabschnitte des Ohrs bzw. Gehörgangs erfassen.

Die Wellenlänge eines Laserstrahls der Lasermessanordnung liegt im blauen Spektralbereich. Dadurch ergibt sich eine geringe Eindringtiefe bzw. eine günstige Streucharakteristik der Haut.

In einer bevorzugten Ausführungsform wird die Laserintensität der Lasermessanordnung in Abhängigkeit von der reflektierten Lichtmenge geregelt. Dadurch können genauere Messungen erzielt werden.

Gemäß einer anderen Weiterbildung können mit der Aufnahmeeinrichtung mehrere vorgegebene optische Merkmale des zu erfassenden Abschnitts erfassbar sein. Dadurch kann beispielsweise die Bewegung der Aufnahmeeinrichtung besser ermittelt und die Einzelaufnahmen mit höherer Genauigkeit aneinander gefügt werden, um ein dreidimensionales Bild zu erhalten.

Weiterhin weist die Aufnahmeeinrichtung eine Beleuchtungseinrichtung zum diffusen Beleuchten des zu erfassenden Abschnitts auf. Durch die eigens vorgesehene Beleuchtungseinrichtung können die optischen Merkmale besser aufgenommen werden.

Vorteilhafterweise ist die Aufnahmeeinrichtung zylindrisch ausgebildet und besitzt einen Durchmesser von weniger als 10 mm, vorzugsweise weniger als 5 mm, so dass sie in einem Gehörgang axial verschiebbar ist. Hierdurch lässt sich eine sehr genaue Abbildung des Gehörgangs gewinnen. Insbesondere ist es dabei von Vorteil, wenn die Aufnahmeeinrichtung vor dem Erfassen in den Gehörgang eingeführt und zum Erfassen aus dem Gehörgang bewegt wird. Anhand des oder der optischen Merkmale werden dann einer oder mehrere Bewegungsvektoren ermittelt, mit deren Hilfe die einzelnen Aufnahmen kombiniert werden.

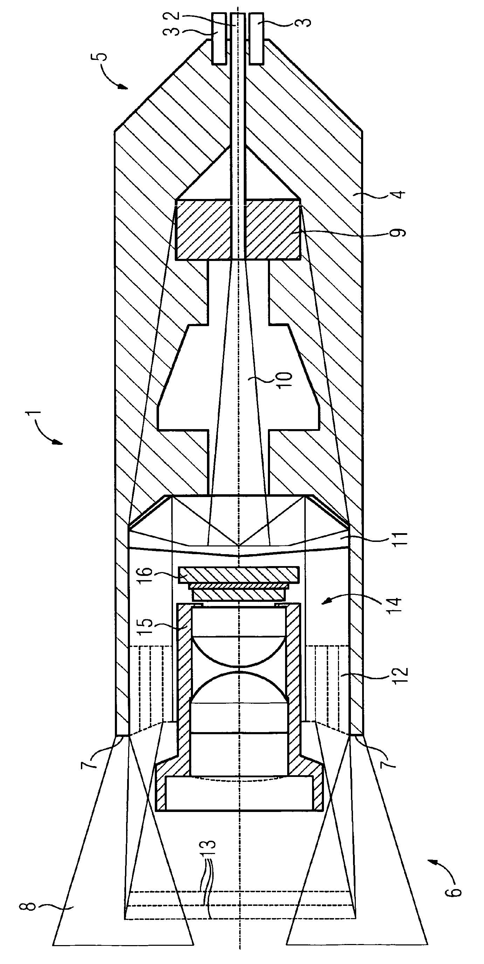

Die vorliegende Erfindung wird nun anhand der beigefügten Zeichnung näher erläutert, die einen Querschnitt durch eine Aufnahmeeinrichtung einer erfindungsgemäßen Vorrichtung zum Erfassen der Gestalt eines Abschnitts des menschlichen Ohrs zeigt.

Die nachfolgend näher geschilderten Ausführungsbeispiele stellen bevorzugte Ausführungsformen der vorliegenden Erfindung dar.

Die in der Figur dargestellte Aufnahmeeinrichtung stellt den optischen Teil einer erfindungsgemäßen Vorrichtung beispielsweise zum Gewinnen von 3D-Daten eines Gehörgangs dar. Die Aufnahmeeinrichtung 1 ist hier zylinderförmig ausgebildet und besitzt etwa einen Durchmesser von wenigen Millimetern (z. B. 2 oder 3 mm). Sie ist damit in einem typischen menschlichen Gehörgang zumindest in axialer Richtung des Gehörgangs frei beweglich. Die Aufnahmeeinrichtung 1 ist im vorliegenden Beispiel mit Hilfe einer Glasfaser 2 an einen Laser angeschlossen, der das Licht für den Messstrahl erzeugt. Weiterhin ist die Aufnahmeeinrichtung beispielsweise mit Kunststofflichtleitern 3 an eine Beleuchtungsquelle angeschlossen, die das Licht zum Erfassen optischer Merkmale in dem Gehörgang liefert. Die Beleuchtungsquelle und der Laser befinden sich im beschriebenen Beispiel außerhalb der Aufnahmeeinrichtung, sie können alternativ aber auch in dieser integriert sein.

Weiterhin besitzt die erfindungsgemäße Vorrichtung eine in der Figur nicht dargestellte Auswerteeinrichtung, um aus gewonnenen Bildern (der Unterabschnitte) 3D-Daten des Gehörgangs zu ermitteln. Die Auswerteeinrichtung ist vorzugsweise über ein elektrisches Kabel (ebenfalls nicht dargestellt) mit der Aufnahmeeinrichtung 1 verbunden. Damit ist die Aufnahmeeinrichtung 1 unabhängig von der Auswerteeinrichtung und gegebenenfalls auch von den Lichtquellen im Gehörgang bewegbar.

Die in der Figur dargestellte Aufnahmeeinrichtung 1 besitzt einen in etwa zylinderförmigen, transparenten Körper 4, der neben einer Lichtleitfunktion auch die Funktion eines Gehäuses übernimmt. Die rechte Stirnseite dieses transparenten Körpers 4 kann als Versorgungsseite 5 bezeichnet werden, in die Licht über die Lichtleiter 3 bzw. die Glasfaser 2 eingespeist wird. Die gegenüberliegende Stirnseite des transparenten Körpers 4 kann als Aufnahmeseite 6 bezeichnet werden, denn an ihr tritt das für die Messungen notwendige Licht aus und es werden reflektierte Strahlen aufgenommen.

Aus den Kunststofflichtleitern 3 wird weißes Licht direkt in einen versorgungsseitigen Abschnitt des transparenten Körpers 4 eingeleitet. Dieser Abschnitt des transparenten Körpers 4 ist so geformt und ausgebildet, dass das Licht in die radial außen liegenden Bereiche des transparenten Körpers in Richtung zur Aufnahmeseite 6 gelenkt wird. An der Aufnahmeseite 6 ist der transparente Körper 4 ringförmig gestaltet, so dass sich für das weiße Licht eine ringförmige Austrittsfläche 7 ergibt. Dort tritt das weiße Licht aus und bildet einen diffus aufgefächerten Beleuchtungsring 8. Er dient zur Beleuchtung der Gehörgangswand, um optische Merkmale wie Flecken, Venen oder Härchen auf der Haut erfassen zu können.

Die Glasfaser 2, die das Laserlicht in die Aufnahmeeinrichtung 1 einbringt, verläuft zunächst durch den versorgungsseitigen Abschnitt des transparenten Körpers 4 hindurch und endet in einer Ferrule 9, welche das Glasfaserende in dem transparenten Körper 4 ausrichtet und fixiert. Die Glasfaser 2 verläuft exakt auf der Achse des transparenten Körpers 4. Der aus dem Glasfaserende austretende Laserstrahl 10 trifft auf ein so genanntes Axikon 11. Dort wird er zu einem ringförmigen Querschnitt aufgeweitet und kollimiert. In dieser Form trifft er auf eine Ring-Projektionslinse 12, die das Laserlicht hier in drei Bündel zerlegt, welche an die Gehörgangswand geworfen werden. Dadurch ergeben sich an der Gehörgangswand drei Laserringe 13.

Eine innerhalb der Ringprojektionslinse 12 angeordnete Kamera 14 besitzt hier ein Weitwinkelobjektiv 15, mit dem reflektiertes Licht in einem weiten Winkel (beispielsweise bis zu 45°) erfasst werden kann. Das erfasste bzw. aufgenommene Licht wird auf einen Kamerachip 16 projiziert. Mit der Kamera 14 sind sowohl optische Merkmale an der Gehörgangswand als auch Reflexionen der Laserringe 13 an der Gehörgangswand aufnehmbar.

Nachfolgend wird die Funktion der Aufnahmeeinrichtung 1 sowie der gesamten erfindungsgemäßen Scanvorrichtung näher dargelegt. Die Scanvorrichtung besitzt, wie erwähnt, die Kamera 14 zur Aufnahme einer Bilderserie. Außerdem verfügt sie über ein Lichtleitsystem, um den Gehörgang zu beleuchten, ein Laserlichtleitsystem und ein optisches Linsensystem 11 und 12 zur Projektion strukturierten Laserlichts (z. B. Ringe 13) an die Gehörgangswand. Hinsichtlich der Erzeugung des strukturierten Lichts wird explizit auf das Dokument PCT/EP 2008/050929 hingewiesen.

Die Laserringe 13 markieren diejenigen Punkte an der Gehörgangswand, für die die Abstandsinformation durch Triangulation gewonnen wird. Durch Bewegen der Aufnahmeeinrichtung aus dem Gehörgang wird die komplette Gehörgangswand gescannt. Die einzelnen durch die Kamera 14 aufgenommenen Bilder werden mit Hilfe einer Lokalisierung natürlicher Merkmale der Gehörgangswand im dreidimensionalen Raum zueinander ausgerichtet. Diese natürlichen bzw. optischen Merkmale können Venen, Hautflecken, die sich farblich von der umgebenden Gehörgangswand unterscheiden, Poren und dergleichen sein. Es kann sich bei diesen Merkmalen aber auch um Härchen handeln, die wie die anderen natürlichen Merkmale im Gehörgang vorhanden sind.

Um die Lokalisierung bzw. Verfolgung der optischen, natürlichen Merkmale zu ermöglichen, wird der Gehörgang diffus beleuchtet, so dass die Kamera 14 diese Merkmale detektieren kann. Die Merkmale werden schließlich mittels Bildverarbeitung detektiert und von Bild zu Bild weiter verfolgt, um einen Bewegungsvektor der Kamera 14 zu erhalten. Mit dieser Information werden die einzelnen Bilder zueinander ausgerichtet, um eine dreidimensionale Punktewolke als Modell des Gehörgangs zu erhalten. Die Anzahl und Qualität der Merkmale wird in einer geeigneten Software ermittelt, und die Lichtmenge in dem Ohrkanal wird entsprechend gesteuert bzw. geregelt.

Die Intensität des Laserstrahls wird in Abhängigkeit von der Schärfe der Laserringprojektion bzw. deren Reflexion geregelt. Bei höherer Laserlichtintensität werden auch deutlich messbare Anteile in tieferen Hautschichten reflektiert, so dass die Schärfe entsprechend reduziert ist. Die Regelung erlaubt daher eine adaptive Verbesserung der Genauigkeit der Einzelaufnahmen bzw. -messungen.

Das gleiche Messprinzip und die gleiche Messvorrichtung können zum Scannen der Ohrmuschel eines menschlichen Ohrs herangezogen werden. In diesem Fall erfolgt die Bewegung der Mess- bzw. Aufnahmeeinrichtung lateral, so dass die projizierten Laserringe alle zu scannenden Abschnitte überfahren.

Eine spezielle Ausführungsform einer erfindungsgemäßen Messvorrichtung kann dadurch gekennzeichnet sein, dass die diffuse Beleuchtung gleichzeitig oder alternierend mit der Laserlichtbestrahlung erfolgt. Entsprechend einem weiteren Ausführungsbeispiel kann die strukturierte Beleuchtung (Laserringe 13) im blauen Spektralbereich erfolgen. Diese blaue Beleuchtung hat Vorteile bezüglich der Eindringtiefe und der Streucharakteristik auf der Haut. Die diffuse Beleuchtung hingegen erfolgt vorteilhafterweise im weißen Spektralbereich, um die natürlichen Merkmale besser erkennen zu können. Falls die Qualität der Merkmalserkennung weiter gesteigert werden soll, kann gegebenenfalls eine Farbkamera als Empfangseinheit eingesetzt werden.

In dem obigen Beispiel erfolgt die Lichterzeugung in einer getrennten Funktionseinheit und die Lichtzuführung über Lichtleitsysteme 2, 3. Alternativ können, wie oben bereits angedeutet, entweder einer oder alle Lichtquellen (Laser, LED) auch in der Aufnahmeeinrichtung selbst enthalten sein.

Eine weitere Verbesserung kann darin bestehen, dass die Frontlinse der Kamera 14 einteilig mit der Ringprojektionslinse gebildet ist. Dieses Bauteil hat dann Beleuchtungs- und Abbildungsfunktion. Der besondere Vorteil dieser Ausführungsform liegt darin, dass die Aufnahmeeinrichtung dann weniger empfindlich gegenüber Verschmutzungen (z. B. Cerumen) ist. Das einteilige optische Element, das die Laserringprojektionslinse und gegebenenfalls eine Fischaugenlinse des Kamerasystems bildet, hat insbesondere auch Vorteile bei der Fertigung und Systemgenauigkeit.

Die Auswerteeinrichtung nutzt vorzugsweise ein Triangulationsverfahren zum Ermitteln der gewünschten Entfernungsinformationen. Dabei wird ausgenutzt, dass die Winkel des Laserstrahls und des Beobachtungsstrahls gegenüber der optischen Achse (Achse der Aufnahmeeinrichtung) und der Abstand der beiden Punkte, in denen der Laserstrahl und der Beobachtungsstrahl die optische Achse schneiden, für die jeweils eingesetzte Aufnahmeeinrichtung bekannt sind.

Der Laserstrahl wird im Gehörgang unterschiedlich gestreut bzw. reflektiert. Die Streulichtcharakteristik hängt von der Gewebeart der Gehörgangswand ab. So kann die Art des Streulichts unmittelbar Auskunft geben, ob hinter der Haut des Gehörgangs Knochenmaterial oder weiches Gewebe liegt. In weichem Gewebe dringt der Laser tiefer unter die Haut als in knöchernen Bereichen. Dies führt also zu unterschiedlichen Beleuchtungsgradienten (d. h. schärfere oder breitere, diffuse Laserringe). Die Unterscheidung dieser Bereiche des Ohrkanals bzw. Gehörgangs mit einer kontaktlosen Messmethode ist für die korrekte Positionierung eines Hörgeräts wichtig. Die Information ist aber auch für die Gestaltung einer Hörgeräteschale bzw. einer Otoplastik nützlich.

Mit der erfindungsgemäßen Messvorrichtung kann somit die dreidimensionale Gestalt eines Ohrkanals oder einer Ohrmuschel ohne den Einsatz von Hilfsmaterial gewonnen werden. Aufgrund der Lokalisation der natürlichen Merkmale muss weder die absolute Position des Messsystems noch die relative Position zum Kopf bekannt sein. Die Position wird lediglich aus den Bildern berechnet, wobei die Eigenschaften des optischen Messsystems bekannt sind. Diese Eigenschaften werden bei der Herstellung durch eine Kalibrationsprozedur ermittelt. Somit ist eine Bewegung des Systems mit der bloßen Hand des Bedieners möglich.

Typische Formen des Trommelfells oder der Ohrmuschel können dazu herangezogen werden, die Kameraorientierung (oben oder unten) zu ermitteln. Dies ist nützlich, um die korrekte Orientierung auf einem Monitor zu erhalten, ohne zu wissen, wie die Kamera in den Gehörgang eingeführt wurde. Dies kann aber auch durch einen Positions- oder Beschleunigungssensor erreicht werden.

Die diffuse Beleuchtung wird im vorliegenden Beispiel dadurch erreicht, dass das Licht von z. B. vier zentralen Fasern zylindrisch um die Kamera geführt wird, so dass an dem Messkopf ein Lichtring gebildet wird. Dies hat den Vorteil, dass verhältnismäßig dicke Lichtleiter mit geringer Dämpfung genutzt werden können. Auch das Laserlicht wird zentral in den Messkopf eingeleitet und rund um die Kamera geführt, um auf die Ringprojektorlinse zu treffen, die die Laserringe erzeugt. Dies erhöht die Stabilität des Messkopfs.

Die Messvorrichtung kann gleichzeitig als Video-Otoskop und Messvorrichtung genutzt werden. Während der Gehörgang betrachtet wird, werden 3D-Daten gesammelt. Auf dem Bildschirm des Video-Otoskops können die gescannten Teile beispielsweise farblich markiert werden. Hierdurch ist es möglich, die Daten des Scans für eine Beratung und die Auswahl eines Hörgeräts zu nutzen, was typischerweise nach einer Otoskopie (Ohrenspiegelung) erfolgt.

## Patentansprüche

1. Vorrichtung zum Erfassen der Gestalt eines Abschnitts eines menschlichen Ohrs, insbesondere eines Gehörgangs, mit
- einer Aufnahmeeinrichtung (1) zum Erfassen einer räumlichen Gestalt eines ersten und mindestens eines zweiten Unterabschnitts des zu erfassenden Abschnitts und
zum Erfassen je einer die Position repräsentierenden Größe des ersten und mindestens zweiten Unterabschnitts relativ zu einem vorgegebenen optischen natürlichen Merkmal des zu erfassenden Abschnitts, wobei
- die Aufnahmeeinrichtung (1) eine Lasermessanordnung (2, 11, 12) aufweist, mit der ein kegelmantelförmiger Messstrahl (13) erzeugbar ist, sowie
- einer Auswerteeinrichtung zum Gewinnen einer Gestaltinformation über den zu erfassenden Abschnitt durch Kombination der Gestalten der Unterabschnitte auf der Basis ihrer erfassten Positionen oder ihrer die jeweilige Position repräsentierenden Größen,
**dadurch gekennzeichnet, dass**
- die Wellenlänge eines Laserstrahls (10) der Lasermessanordnung (2, 11, 12) im blauen Spektralbereich liegt, und die Aufnahmeeinrichtung (1) eine Beleuchtungseinrichtung (3, 4, 7) zum diffusen Beleuchten des zu erfassenden Abschnitts im weißen Spektralbereich aufweist.

2. Vorrichtung nach Anspruch 1, wobei mit der Lasermessanordnung (2, 11, 12) gleichzeitig mehrere kegelmantelförmige Messstrahle (13) erzeugbar sind.

3. Vorrichtung nach Anspruch 1 oder 2, wobei die Laserintensität der Lasermessanordnung (2, 11, 12) in Abhängigkeit von der reflektierten Lichtmenge geregelt wird.

4. Vorrichtung nach einem der vorhergehenden Ansprüche, wobei mit der Aufnahmeinrichtung (1) mehrere vorgegebene optische Merkmale des zu erfassenden Abschnitts erfassbar sind.

5. Vorrichtung nach einem der vorhergehenden Ansprüche, wobei die Aufnahmeeinrichtung (1) zylindrisch ausgebildet ist und einen Durchmesser von weniger als 5 mm sowie einer Länge kleiner 15 mm besitzt, so dass sie in einem Gehörgang axial verschiebbar ist.

6. Verfahren zum Erfassen der Gestalt eines Abschnitts eines menschlichen Ohrs, insbesondere eines Gehörgangs,
durch
- Erfassen einer räumlichen Gestalt eines ersten und mindestens eines zweiten Unterabschnitts des zu erfassenden Abschnitts mit Hilfe eines Laserstrahls (10) und
- Erfassen je einer die Position repräsentierenden Größe des ersten und mindestens zweiten Unterabschnitts relativ zu einem vorgegebenen optischen natürlichen Merkmal des zu erfassenden Abschnitts, sowie
- Gewinnen einer Gestaltinformation über den zu erfassenden Abschnitt durch Kombination der Gestalten der Unterabschnitte auf der Basis ihrer erfassten Positionen oder ihrer die jeweilige Position repräsentierenden Größen,
**dadurch gekennzeichnet, dass**
- das Erfassen der räumlichen Gestalt mit Hilfe eines Laserstrahls (10) im blauen Spektralbereich erfolgt und
- das Erfassen je einer die Position repräsentierenden Größe mit Hilfe diffuser Beleuchtung des zu erfassenden Abschnitts im weißen Spektralbereich erfolgt.

7. Verfahren nach Anspruch 6, wobei die Gestalt eines Gehörgangs und mindestens ein optisches Merkmal in dem Gehörgang mit einer Aufnahmeeinrichtung (1) erfasst wird, indem die Aufnahmeeinrichtung (1) vor dem Erfassen in den Gehörgang eingeführt und zum Erfassen aus dem Gehörgang bewegt wird.

## Claims

1. Device for recording the form of a section of a human ear, especially of an auditory canal, with
- a recording device (1) for recording a spatial form of a first and of at least one second subsection of the section to be recorded and
for recording a variable representing a position in each case of the first and at least second subsection relative to a predetermined optical natural feature of the section to be recorded, with
- the recording device (1) having a laser measuring arrangement (2, 11, 12) with which a cone-surface-shaped measuring beam (13) is able to be generated, and also
- an evaluation device for obtaining information about the form of the section to be recorded by combining the forms of the subsections based on their recorded position or their variables representing the respective position,
**characterised in that**
- the wavelength of a laser beam (10) of the laser measuring arrangement (2, 11, 12) lies in the blue area of the spectrum, and the recording device (1) has an illumination device (3, 4, 7) for diffuse illumination of the section to be recorded in the white area of the spectrum.

2. Device according to claim 1, with a number of cone-surface-shaped measuring beams (13) able to be generated simultaneously with the laser measuring arrangement (2, 11, 12) .

3. Device according to claim 1 or 2, with the laser intensity of the laser measuring arrangement (2, 11, 12) being regulated as a function of the reflected amount of light.

4. Device according to one of the previous claims, with a number of predetermined optical features of the section to be recorded able to be recorded with the recording device (1).

5. Device according to one of the previous claims, with the recording device (1) being embodied cylindrically and possessing a diameter of less than 5 mm and also a length smaller than 15 mm, so that it is able to be pushed axially into an auditory canal.

6. Method for recording the form of a section of the human ear, especially of an auditory canal,
by
- recording a spatial form of a first and at least one second subsection of the section to be recorded with the aid of a laser beam (10) and
- recording a variable representing the position in each case of the first and at least second subsection relative to a predetermined optical natural feature of the section to be recorded, and also
- obtaining form information about the section to be recorded by combination of the shapes of the subsections on the basis of their recorded positions or their variables representing the respective position,
**characterised in that**
- the spatial form is recorded with the aid of a laser beam (10) in the blue area of the spectrum and
- the variable representing the position in each case is recorded with the aid of diffuse illumination of the section to be recorded in the white area of the spectrum.

7. Method according to claim 6, with the form of an auditory canal and at least one optical feature in the auditory canal being recorded with a recording device (1), in that the recording device (1) is introduced prior to the recording into the auditory canal and is moved out of the auditory canal to make the recording.

## Revendications

1. Dispositif de relevé de la forme d'une partie d'une oreille humaine, notamment d'un tuyau auditif, comprenant
- un dispositif ( 1 ) d'enregistrement pour le relevé d'une forme spatiale d'une première et d'au moins une deuxième sous-parties de la partie à relever et pour le relevé respectivement d'une grandeur représentant la position de la première et d'au moins la deuxième sous-parties par rapport à une caractéristique optique naturelle prescrite de la partie à relever, dans lequel
- le dispositif ( 1 ) d'enregistrement comporte un agencement ( 2, 11, 12 ) de mesure laser, par lequel un faisceau ( 13 ) de mesure en forme de surface latérale de cône peut être produit, ainsi que
- un dispositif d'exploitation pour l'obtention d'une information de forme sur la partie à relever par combinaison des formes des sous-parties sur la base de leurs positions relevées ou de leurs grandeurs représentant la position respective,
**caractérisé en ce que**
- la longueur d'onde d'un faisceau (10) laser de l'agencement ( 2, 11, 12 ) de mesure laser est dans le domaine bleu du spectre, et le dispositif ( 1 ) d'enregistrement a un dispositif ( 3, 4, 7 ) d'éclairage pour l'éclairage diffus de la partie à relever dans le domaine blanc du spectre.

2. Dispositif suivant la revendication 1, dans lequel plusieurs faisceaux (13) de mesure en forme de surface latérale de cône peuvent être produits en même temps par l'agencement ( 2, 11, 12 ) de mesure laser.

3. Dispositif suivant la revendication 1 ou 2, dans lequel l'intensité laser de l'agencement (2, 11, 12) de mesure laser est réglée en fonction de la quantité de lumière réfléchie.

4. Dispositif suivant l'une des revendications précédentes, dans lequel plusieurs caractéristiques optiques prescrites de la partie à relever peuvent être relevées par le dispositif ( 1 ) d'enregistrement.

5. Dispositif suivant l'une des revendications précédentes, dans lequel le dispositif (1) d'enregistrement est cylindrique et a un diamètre de moins de 5 mm ainsi qu'une longueur plus petite que 15 mm, de manière à pouvoir coulisser axialement dans un tuyau auditif.

6. Procédé de détection de la forme d'une partie d'une oreille humaine, notamment d'un tuyau auditif, dans lequel,
- on relève une forme dans l'espace d'une première et d'au moins une deuxième sous-parties de la partie à relever à l'aide d'un faisceau ( 10 ) laser et
- on relève respectivement une grandeur, représentant la position, de la première et de la au moins une deuxième sous-parties par rapport à une caractéristique naturelle optique prescrite de la partie à relever, ainsi que
- on obtient une information de forme sur la partie à relever par combinaison des formes des sous-parties sur la base de leurs positions relevées ou de leurs grandeurs représentant la position respective,
**caractérisé en ce que**
- on effectue le relevé de leurs formes dans l'espace à l'aide d'un faisceau ( 10 ) laser dans le domaine bleu du spectre et
- on effectue la détection de respectivement une grandeur représentant la position à l'aide d'un éclairage diffus de la partie à relever dans le domaine blanc du spectre.

7. Procédé suivant la revendication 6, dans lequel on relève la forme d'un tuyau auditif et au moins une caractéristique optique dans le tuyau auditif par un dispositif (1) d'enregistrement en introduisant le dispositif (1) d'enregistrement avant le relevé dans le tuyau auditif et en le sortant du tuyau auditif pour le relevé.
